(19) Europäisches Patentamt / European Patent Office / Office européen des brevets

(11) **EP 3 877 045 B1**

(12) **EUROPEAN PATENT SPECIFICATION**

(45) Date of publication and mention
of the grant of the patent:
**30.07.2025 Bulletin 2025/31**

(21) Application number: **19883311.3**

(22) Date of filing: **04.11.2019**

(51) International Patent Classification (IPC):
*A61N 1/365* (2006.01)   *A61B 5/00* (2006.01)
*A61B 5/022* (2006.01)   *A61B 5/021* (2006.01)
*A61B 5/0215* (2006.01)

(52) Cooperative Patent Classification (CPC):
**A61N 1/36564;** A61B 5/021; A61B 5/0215;
A61B 5/022; A61B 5/4836

(86) International application number:
**PCT/US2019/059703**

(87) International publication number:
**WO 2020/096982 (14.05.2020 Gazette 2020/20)**

(54) **TREATMENT OF DRUG RESISTANT HYPERTENSION ASSOCIATED WITH IMPAIRED LEFT VENTRICULAR FUNCTION AND BRADYCARDIA USING A CARDIAC PACEMAKER**

BEHANDLUNG VON ARZNEIMITTELRESISTENTER HYPERTONIE ASSOZIIERT MIT BEEINTRÄCHTIGTER LINKSVENTRIKULÄRER FUNKTION UND BRADYKARDIE UNTER VERWENDUNG EINES HERZSCHRITTMACHERS

TRAITEMENT DE L'HYPERTENSION PHARMACORÉSISTANTE ASSOCIÉE À UNE FONCTION VENTRICULAIRE GAUCHE ALTÉRÉE ET À UNE BRADYCARDIE À L'AIDE D'UN STIMULATEUR CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2018 US 201862757559 P**
**12.04.2019 US 201962833052 P**

(43) Date of publication of application:
**15.09.2021 Bulletin 2021/37**

(73) Proprietor: **BaroPace, Inc.**
**Ashland, OR 97520 (US)**

(72) Inventor: **BURNAM, Michael**
**Ashland, Oregon 97520 (US)**

(74) Representative: **Eisenführ Speiser**
**Patentanwälte Rechtsanwälte PartGmbB**
**Johannes-Brahms-Platz 1**
**20355 Hamburg (DE)**

(56) References cited:
| | |
|---|---|
| **US-A- 4 791 931** | **US-A- 5 129 394** |
| **US-A- 6 141 590** | **US-A1- 2007 299 477** |
| **US-A1- 2007 299 477** | **US-A1- 2009 036 940** |
| **US-A1- 2016 220 824** | **US-A1- 2016 220 824** |
| **US-A1- 2017 304 048** | **US-A1- 2017 304 048** |
| **US-B2- 8 046 070** | |

**Description**

Field of the Technology

The invention relates to an apparatus for treating diastolic heart failure and for controlling blood pressure in patients, who have proven to be resistant to drug treatments for blood pressure control.

**Description of the Prior Art**

**Hypertension is the angle largest contributor to cardiovascular death. It dramatically increases risk of heart attack, stroke, heart failure, and kidney failure. The annual direct costs of hypertension are estimated at $500 billion worldwide. Almost 20 percent of patients are completely non-adherent to oral medications while nearly half are partially non-**adherent, highlighting the need for alternative treatment options.

Hypertension causes increased systemic vascular resistance (SVR) and vice versa. SVR is calculated by subtracting the right atrial pressure (RAP) or central venous pressure (CVP) from the mean arterial pressure (MAP), divided by the cardiac output and multiplied by 80. Normal SVR is 700 to 1500. RAP can be measured by a pacemaker with the appropriate sensor. Non-invasive and invasive measurements of cardiac output exist.

The end result of long-standing drug resistant hypertension (DRH) is diastolic congestive heart failure (DCHF) or heart failure with preserved ejection traction (HFpEF), which is defined as the amount of Blood pumped put with each heart beat expressed as a percentage. DCHF most commonly results from left ventricular thickening (hypertrophy) and stiffness (diastolic dysfunction) caused by sub-optimally treated or drug resistant hypertension. Six million patients in the US, and twenty-three million patients, worldwide suffer from congestive heart failure. Forty percent of those patients have DCHF which is the eighth most common reason for hospital admission and represents 3% of total healthcare costs in the US and Europe. The total cost of such care in the US in 2017 was $30 billion, and it is projected to exceed $50 billion by 2030. Resistant hypertension is defined as blood pressure (hat remains above goal (American Heart Association Guidelines or other accepted criteria appropriate by virtue of demographics and geography) despite concurrent use of three antihypertensive agents of different classes, one of which should be a diuretic. Patients whose blood pressure is controlled with four or more medications are also considered to have resistant hypertension. Patients with resistant hypertension are at high risk for adverse cardiovascular events (the development of heart failure, myocardial infarction, arrhythmia, stroke, death or renal failure) and are more likely than those with controlled hypertension to have a secondary cause, which is usually at least in part reversible.

Two prior attempts to treat drug resistant hypentension (DRH) using devices have failed. Medtronics Inc. announced the results of its SPYRAL HTN trial in 2018, a head-to-head evaluation of invasive Renal Denervation in 433 patients. Renal denervation was not effective in the treatment of DRH and the technology is no longer in use. Carotid sinus electrical stimulation sponsored by the US pharmaceutical company CVRx (BAROS TIM NEO), another invasive method targeting the sympathetic nervous system, was also ineffective (2012) and is no longer in use. Moreover, neither technology showed any promise for the treatment of DCHF.

Bradycardia is defined as a condition wherein an individual has a slow heart rate. typically defined as a heart rate of under 60 beats per minute (BPM) in adults. Bradycardia typically does not cause symptoms until the rate drops below 50 BPM. When symptomatic, it may cause fatigue, weakness, dizziness, swcating, and at very low rates, fainting. During sleep, a slow heartbeat with rates between 40-50 BPM is common, and is considered normal. Highly trained athletes may also have athletic hean syndrome, a very slow resting heart rate that occurs as a sport adaptation and helps prevent tachycardia during training. The term relative bradycardia is used in explaining a heart rate that, although not actually below 60 BPM, is still considered too slow for the individual's current medical condition or causes symptoms such as weakness, dizziness, or fainting.

Sinus node dysfunction refers to the condition in which a patient experiences an abnormality in the heartbeat or experiences arrhythmias (irregular heart beats) due to a malfunction of the sino atrial node or the sinus node. The sinus node is where the electrical pulse, which initiates the pumping action of the heart, originates. The earliest known version of the condition was known as "sick sinus syndrome" and today it refers to the abnormalities arising in the formation of the pulse in the sinus node and its propagation, namely conditions like sinus bradycardia, sinus pause, chronotropic incompetence and sinoatrial exit block. Sinus node dysfunction is a disease primarily associated with the elderly. It is mainly caused by the organic process of aging of the sinus node, but can also be the result of heart attack, inflammation, other forms of tissue loss or drugs..

Chronotropic incomperance (CI). which is one of the forms of sinus node dysfunction. is broadly defined as the inability of the heart to increase its rate commensurate with increased activity or demand, is common in patients with cardiovascular disease, produces exercise intolerance which impairs quality-of-life, and is an independent predictor of major adverse cardiovascular events and overall mortality. Chronotropic incompetence (CI) is most commonly diagnosed when the heart rate (HR) fails to reach an arbitrary percentage, typically 85%, 80%, or less commonly, 70% depending upon the guidelines in use, of the age-predicted maximum heart rate (APMHR), which is usually based on the simple equation. 220 - age in years, obtained during an incremental dynamic exercise test. CI is usually diagnosed during maximal exercise, most commonly assessed during a graded treadmill exercise test. CI can also be determined by the HR reserve, which is the change in HR from rest to peak exercise during an exercise test.

Relative bradycardia is herein defined as a persistent heart rate less than 60 beats per minute and greater than 40 beats per minute.

An atrial pacemaker (AP) is an apparatus that sends electrical impulses to the right or left atrium of the heart when intact atrio-ventriuclar (AV) node conduction is present in order to set the heart rhythm.

A dual chamber pacemaker (DCP) is an apparatus that sends electrical impulses to either the right or left atrium, or to the right ventricle of the heart in the presence of intact or abnormally reduced AV nodal conduction in order to set the rhythm of the heart.

An automatic implanted cardiac defibrillator (AICD) is an apparatus which is capable of shocking the heart after the detection of certain arrhythmias that is not designed to pace the cardiac atrium producing normally activated cardiac contraction.

A combined-pacemaker/AICD is an implantable cardiac device that combines the features of an AICD with a dual chamber pacemaker. This permits both standard atrio-ventricular synchronized pacing for sinus node dysfunction and the detection and reversion by defibrillation of serious cardiac arrhythmias.

A CRT bi-ventricular pacemaker is a traditional pacemaker used to treat slow heart rhythms. Pacemakers regulate the right atrium and right ventricle to maintain a good heart rate and keep the atrium and ventricle working together. This is called AV synchrony. Biventricular pacemakers add a third lead to help the left ventricle contract. RV pacing alters the normal synchrony of the heart which begins in the LV, not the RV, thus reversing normal or physiologic heart function. LV pacing can restore normal synchrony. If a patient with SHF has very slow intraventricular conduction, RV pacing further aggravates this and can worsen or precipitate heart failure. (Pacing via an LV lead restores normal activation. Ventricular sequence pacing is programmable in most modem devices. Heart failure with reduced ejection fraction (HFrEF), also called systolic failure (SHF) is where the left ventricle loses its ability to contract normally. The heart can't pump with enough force to push enough blood into circulation.... The heart can't properly fill with blood during the resting period between each heat.

A CRT-D bi-ventricular pacemaker with AICD is a bi-ventricular pacemaker combined with an AICD.

Conventional guidelines for a pacemaker implant are generally based on symptoms, the presence of heart disease and the presence of symptomatic bradyarrhythmias. Pacemakers for tachyarrhythmias, cardioversion and defibrillation are also available. For example, the American Heart Association Guidelines used in the U.S divide indications for permanent pacing in sinus node dysfunction into two classes. Class 1 is defined as:

1. Sinus node dysfunction with documented symptomatic bradycardia. including frequent sinus pauses that produce symptoms. In some patients, bradycardia is iatrogenic and will occur as a consequence of essential long-term drug therapy of a type and dose for which there are no acceptable alternatives. (Level of evidence: C)

2. Symptomatic chronotropic incompetence. (Level of evidence: C)

Class IIa is defined as:

1. Sinus node dysfunction occurring spontaneously or as a result of necessary drug therapy with heart rate <40 beats per minute (bpm) when a clear association between significant symptoms consistent with bradycardia and the actual presence of bradycardia has not been documented. (Level of evidence: C)

Class 11b is defined as:

1. In minimally symptomatic patients, chronic heart rate <30 bpm while awake. (Level of evidence: C)

Class III is defined as:

1. Sinus node dysfunction in asymptomatic patients. including those in whom substantial sinus bradycardia (heart rate <40 bpm) is a consequence of long-term drug treatment.

2. Sinus node dysfunction in patients with symptoms suggestive of bradycardia that are clearly documented as not associated with a slow heart rate.

3. Sinus node dysfunction with symptomatic bradycardia due to nonessential drug therapy.

Systolic heart failure heart failure is defind as severely reduced LV function, usually left ventricular ejection fraction (LVEF) < 35%. This is the so-called forward heart failure, or severely impaired LV contraction.

Diastolic heart failure or clinical heart failure in the presence of a normal LVEF or HFpEF associated with impaired LV relaxation is also called reverse heart failure, or heart failure with a normal ejection fraction.

Combined heart failure is defined as the presence of both systolic and diastolic heart failure in the same patient.

In a 61-country study conducted by the World Society of Arrhythmias, there were a total of 1,002,664 pacemakers counted. The United States has the largest number of patients with internal cardiac pacemakers, totaling 225,567. In 1991, the National High Blood Pressure Education Program (NHBPEP) estimated 43.3 million adults had hypertension in United States. [1] Hypertension was defined as systolic blood pressure (SBP) equal to or greater than 140 mm Hg and diastolic BP (DBP) as equal or more than 90 mm Hg or defined as those taking medication for hypertension. The number of patients estimated to have severe hypenension defined as a systolic blood pressure equal to or greater than 165 and a diastolic BP equal to or greater than 105 is estimated at 3.5 million adults. The incidence of sino-atrial node dysfunction in the US severe enough to warrant a pacemaker in adults age 50 or older is 0.8 per 1000.

The Providence cohort had 70,000 patients, and 2200 had pacemakers or 3.1 %. By **extrapolation of the data, if patients in the Providence cohort with DRH and Sick Sinus** Syndrome, the latter not necessarily severe enough to warrant a pacemaker had received a pacemaker implant to treat DRH as the primary indication, the number of pacemakers would have increased to 12%. This would significantly increase the market for pacemakers **worldwide.**

US 2016 0220 824 A1 discloses a method that electrically stimulates a heart muscle to alter the ejection profile of the heart, to control the mechanical function of the heart and reduce the observed blood pressure of the patient. The therapy may be invoked by an implantable blood pressure sensor associated with a pacemaker like device. In some cases, where a measured pretreatment blood pressure exceeds a treatment threshold, a patient's heart may be stimulated with an electrical stimulus timed relative to the patients cardiac ejection cycle. This is done to cause dyssynchrony between at least two cardiac chambers or within a cardiac chamber, which alters the patients cardiac ejection profile from a pretreatment cardiac ejection profile. This has the effect of reducing the patients blood pressure from the measured pretreatment blood pressure.

US 2009 036 940 A1 discloses a method for sensing a pulmonary artery pressure (PAP) and providing a sensed PAP signal, detecting an abnormal blood pressure (BP) condition using information from the sensed PAP signal, delivering a pacing energy to a heart, and automatically altering at least one pacing characteristic in response to the detected abnormal BP condition. The detecting an abnormal BP condition can include detecting various forms of hypertension or hypotension. The automatically altering the at least one pacing characteristic can include automatically altering at least one of a pacing rate, a pacing waveform, an atrioventricular (AV) delay, an interventricular (VV) delay, a pacing mode, or a pacing site. The method can also include delivering vagal nerve stimulation and automatically altering the vagal nerve stimulation in response to the detected abnormal BP condition. The detecting the abnormal BP condition can also include using a sensed auxiliary physiological parameter.

## Brief Summary

The combination of bradycardia and impaired left ventricular (LV) stroke volume may be seen in patients with drug resistant hypertension that can be corrected by the implantation of a permanent cardiac pacing device, which is utilized as disclosed below. This represents a new indication for pacemaker implantation and also justifies modifying existing guidelines for pacemaker and/or cardio-defibrillator implants.

Moreover, because impaired LV function results in heart failure (systolic and diastolic), it follows that optimization of peripheral resistance by pacemaker therapy in the presence of bradycardia, and possibly in those without bradycardia but with severe LV dysfunction, will enhance the non-pharmacologic treatment of both systolic and diastolic heart failure.

Current generation pacemakers provide only heart rate-based modulation. Sensors inside the pacemaker, such as accelerometers and respiratory movement detectors, regulate pacemaker-mediated heart rate according to preprogrammed heart rate profiles. NO existing pacemaker type in clinical use is also regulated by blood pressure.

The invention is defined in independent claim 1. Further embodiments are disclosed in dependent claims 2-12. References to methods below are not part of the claimed invention as such, but are useful for the general understanding of the invention.

Based upon the clinical observations presented, regulation of pacemaker function by blood pressure promises to better treat DRH and DCHF. This can be accomplished by integrating a real-time blood pressure measurement device, such as a wristband sensor now generally available, linked via encrypted blue-tooth connectivity to a standard dual chamber pacemaker containing the software described herein. The software program is tailored to the patient's needs by the supervising physician via external programmability with access to real-time blood pressure data received from the patients blood pressure sensor. The software calculates optimal pacemaker function to better treat DRH and DCHF. The software of the illustrated embodiments could be resident in the blood pressure cuff, a smart phone or other separate peripheral device such as the standard doctor's office programmers currently in use, or the pacemaker. A different data loop integrates a blood pressure cuff sending data over the internet or phone to a distant processing site, and then the new pacemaker instructions arriving again via the internet or the phone. Below is a disclosure of various permutations and three embodiments.

No method previously or currently exists to interface a cardiac pacing device with an external blood pressure measuring device for the purpose of regulating pacemaker function for any purpose. Similarly, no automatic software-driven feedback loops are available linking an implanted cardiac pacemaker to a blood pressure measuring device, where the loop also integrates a software program to interact with the pacemaker in a manner that regulates cardiac pacing to control blood pressure and/or treat diastolic congestive heart failure.

We have shown using the data from two retrospective clinical trials that implantation of a dual chamber pacemaker for standard indications in patients with drug resistant hypertension

(DRH) and DRH with diastolic congestive heart failure (DCHF) reduces blood pressure, the magnitude of drug therapy needed for optimal blood pressure regulation, and further occurrences of DCHF. This data also showed a correlation between the drop in SBP and the percentage of right atrial (RA) pacing between RA pacing percentages of 0 to 40% or such other percentage range consistent with the teachings of this invention and as may later be determined by clinical experience. Therefore, whenever the pacing percentage of 40% is indicated in this specification, if should be understood that this parameter may be changed without departing from the scope of the invention, as defined by the appended claims. The systolic blood pressure reduction data between 0 and 40% changes in right atrial pacing rate follows a polynomial distribution typical of physiologic data. For the clinical cohort analyzed, no further drop in SBP reliably occurred above an increase in RA pacing of 40% although this drop is expected to change in different clinical cohorts. Moreover, the data approaches linearity between 0 and 40% increases in RA pacing rates providing an equation that relates the expected drop in SBP for each incremental increase in RA Pacing. These clinical findings and the described mathematical relationship form the basis of a new method to treat both DRH and DRH with DCHF.

A software program can be written that links the pacemaker and an external or internal measurement of blood pressure. One embodiment utilizes a wristwatch-type BP monitor now generally available with blue tooth connectivity worn by the patient. The software allows either clinician-directed programming of the pacemaker's blood pressure algorithm through the use of an external programmer with access to any real-time blood pressure measurements, or direct (blue tooth connectivity) to the blood pressure algorithm resident in the pacemaker's internal processor. Together, these two types of pacemaker regulation by an algorithm linked to the measurement of blood pressure represent new treatment options for drug resistant hypertension and diastolic congestive heart failure.

What has been developed is an upgraded or modified method of operation of virtually all types of pacemakers that allows the pacing device to automatically, or by the physician to remotely tailor HR to blood pressure. This type of operation is defined in this application as "Blood Pressure Adaptive Pacing" (BPAP), which optimizes not only blood pressure but peripheral resistance in patients with DRH, heart failure (systolic and diastolic) or the combination of both. Blood Pressure Adaptive Pacing is realized in multiple embodiments.

One approach is to externally program the pacing device in the cardiologist's office using available blood pressure data through an external interface. The components of such a system is envisioned as including the pacing device, an onboard computer control and memory for storing the algorithm in the pacing device, a computer interface in the physican's office to connect the pacemaker either wirelessly or by hardwired sensor placed over the chest similar to that employed for pacemaker evaluation in the physician's office or clinic.

Another approach envisions remotely inputting the patient's electronic blood pressure measurements obtained outside the physician's office as sent wirelessly to the patient's pacemaker via an external wireless interface present in the patient's home.

Yet another approach envisions direct in vivo sensing carried out by the patient's implanted cardiac device using appropriate software on an independent dynamic basis. An in vivo blood pressure is included in or with the pacemaker. The pacemaker is programmed, monitored, and adjusted in the physician's office or clinic to operate according the disclosed methodologies,

It can now be appreciated that the illustrated embodiments of the invention include an apparatus employing a new algorithm for pacing in the right atrium for the purpose of reducing blood pressure in patients having drug resistant hypertension and to patients with diastolic congestive heart failure using real time feedback by monitoring blood pressure, biological markers or other vital signs in which the normal synchronicity of the heart is maintained.

The apparatus and a non-claimed method employs a Bluetooth enabled wristwatch for monitoring the blood pressure, biological markers or other vital signs and generating a control signal to a right atrial implanted pacemaker. It is a cardiac pacing device that permits RA pacing via an implanted lead in the RA.

The apparatus and non-claimed method further include means for releasing atrial naturetic peptides.

Thus, it can be appreciated that the illustrated embodiments include an apparatus which has a programmable, implantable pacemaker with a controllable pacing rate; and a blood pressure monitoring device having an output communicated to the pacemaker. The pacemaker selectively and automatically modulates pacing rate in response to monitored blood pressure to reduce hypertensive blood pressure in a patient.

In one embodiment the pacemaker is a RA pacemaker or more properly a cardiac pacing device that can pace the RA via an implanted lead, and where the blood pressure monitoring device measures peripheral blood pressure.

The blood pressure monitoring device includes any known type of blood pressure sensor or cuff, such as: a pneumatic cuff relying on mechanical compression of a peripheral artery, most commonly the brachial artery in the arm but can also be used on the ankle or the wrist; a non-pneumatic cuff which analyzes the arterial waveform and function anywhere on the body where the arterial pulse contour can be sensed, most commonly at the wrist: and an implantable sensor within blood vessels or the heart chambers.. The cuffless BP monitors now being FDA approved function by processing the arterial waveform which can be obtained at multiple sites on the body, including the earlobe, any digit. The implanted sensor is implanted at a vascular site or a cardiac site.

The blood pressure monitoring device communicates wirelessly with the pacemaker, such as through Bluetooth technology.

The blood pressure monitoring device may further include a pulse oximeter, and/or a chemical sensor for sensing glucose, electrolytes or other blood parameters.

The blood pressure monitoring device monitors systolic blood pressure or may be configured to monitor diastolic or systolic blood pressure or mean arterial pressure. The device may also be connected to a separate apparatus that measures cardiac stroke volume (such as ultrasound) and therefore calculates systemic vascular resistance. .

The illustrated embodiments also extend to a non-claimed method for operating a pacing device including the steps of:

activating a systolic blood pressure monitor coupled to a patient, storing a number of systolic blood pressure readings; determining a baseline systolic blood pressure reading; selecting the following parameters for use in a pacemaker for blood pressure regulation, namely a target SBP (systolic blood pressure), a lower limit of acceptable SBP; a target treatment interval in minutes; and/or target pacing rate change per treatment interval where the pacing rate change ranges from 0 - 40%;monitoring systolic blood pressure; if systolic blood pressure exceeds the target SBP, using a pacemaker having a pacing rate to treat the patient by: increasing the pacing rate of the pacemaker by either a default level of 5% per treatment, or by a different predetermined value; monitoring the SBP for a predetermined time period to establish the new blood pressure baseline; comparing SBP to a pre-selected optimal SBP; increasing the pacing rate of the pacemaker by a predetermined incremental amount; and repeating the steps of comparing SBP and increasing the pacing rate of the pacemaker until either the SBP falls to the target SBP, or the pacing rate of the pacemaker exceeds a predetermined maximal value.

While the illustrated embodiment has been disclosed in terms of systolic BP and RA pacing rtes between 0 and 40%, it is within the scope of the invention to also use diastolic or mean BP and other RA pacing rates.

The illustrated embodiments also extend to blue tooth regulation of the pacemaker pacing function generated by a smart phone in which the software of the illustrated embodiments has been installed.

In the illustrated method the pacing rate of the pacemaker is a RA pacing rate.

Again monitoring blood pressure includes monitoring peripheral blood pressure, intravascular blood pressure or intracardiac blood pressure.

The step of monitoring peripheral blood pressure includes the step of monitoring peripheral blood pressure with a wrist mounted device or ann cuff.

The non-claimed method may further include monitoring blood oxygen levels, glucose levels, blood electrolytes levels or other blood parameters and controlling the pacing rate in response to the monitored blood oxygen levels, glucose levels, blood electrolytes levels or other blood parameters.

In one embodiment the pacing device is a RA pacemaker, and selecting the following parameters for use in a pacemaker for blood pressure regulation includes selecting a target RA pacing rate change per treatment interval where the RA pacing rate change ranges from 0 - 40%. If systolic blood pressure exceeds the target SBP. use of a pacemaker having a RA pacing rate to treat the patient is made. Treating the patient increases the RA pacing rate of the pacemaker by either a default level of 5% per treatment, or by a different predetermined value. Increasing the pacing rate of the pacemaker by a predetermined incremental amount increases the RA pacing rate. Repeating the steps compares SBP and increases the RA pacing rate of the pacemaker until either the SBP falls to the target SBP, or the RA pacing rate of the pacemaker exceeds a predetermined maximal value. Another possible pacing parameter could be the duration of RA pacing. For example, sense the SBP, raise the RA pacing 5% for ten minutes where the ten minutes could be preprogrammed overriding the sample and treat every five minutes idea.

The disclosure also include non-claimed method for operating a pacing device to treat drug resistant hypertension including the steps of: monitoring blood pressure; and controlling heart rate in the pacing device in response to the monitored blood pressure to selectively prevent excessive pacing to reduce mean arterial blood pressure by either inhibiting heart rate in the pacing device or by changing heart rate parameters.

The step of changing rate modulation parameters includes changing acceleration of pacing rate including magnitude of acceleration, and/or duration of acceleration, and changing deceleration of pacing rate including magnitude of deceleration, and/or duration of deceleration.

The scope of the invention includes using the disclosed algorithm and measured BP to better regulate standard rate modulation. Current rate modulation software, particularly in the elderly, is often detrimental or high activity levels, such as treadmill exercise testing. There is reason to believe that exercise performance in the elderly, in patients with DRH, and patients with DCHF will be enhanced when rate modulation software is further regulated by the addition of the disclosed software.

The step of monitoring blood pressure in one embodiment includes the step of monitoring systolic blood pressure, and the step of controlling rate modulation in the pacing device in response to the monitored blood pressure includes the step of controlling rate modulation in the pacing device in response to the monitored systolic blood pressure to selectively prevent excessive pacing to reduce mean systolic arterial blood pressure by either inhibiting rate modulation in the pacing device or by changing rate modulation parameters.

The step of monitoring blood pressure monitors diastolic blood pressure; and the step of controlling rate modulation in the pacing device in response to the monitored blood pressure controls rate modulation in the pacing device in response to the monitored diastolic blood pressure to selectively prevent excessive pacing to reduce mean diastolic arterial blood pressure by either inhibiting rate modulation in the pacing device or by changing rate modulation parameters.

The non-claimed further includes the step of monitoring blood oxygen levels, noninvasive measurement of pulse oximetry, glucose levels, blood electrolytes levels or other blood parameters and controlling the pacing rate in response to the monitored blood oxygen levels, glucose levels, blood electrolytes levels or other blood parameters.

The step of monitoring blood pressure includes monitoring peripheral blood pressure, intravascular blood pressure or intracardiac blood pressure.

The disclosure can be better visualized by turning now to the following drawings wherein like elements are referenced by like numerals.

## Brief Description of the Drawings

Fig. 1 is diagram illustrating the programming of the pacemaker with the blood pressure monitor using an external programming device.

Fig. 2 is a flow diagram of the programming steps used in the diagram of Fig. 1.

Fig. 3 is a flow diagram of the programming of the communication of the blood pressure monitor with the pacemaker.

Fig. 4 is a diagram of the feedback control of the treatment algorithm of one of the illustrated embodiments.

Fig. 5 is a flow diagram showing the monitor-only mode of operation.

Fig. 6 is a flow diagram of an embodiment of the treatment algorithm.

The disclosure and its various embodiments can now be better understood by turning to the following detailed description of the preferred embodiments which are presented as illustrated examples of the embodiments defined in the claims. It is expressly understood that the embodiments as defined by the claims may be broader than the illustrated embodiments described below.

## Detailed Description of the Preferred Embodiments

Hypertension increases incrementally with aging. Heart Rate incrementally decreases with aging. While multiple factors combine to cause hypertension to develop and progress with aging, including but not limited to atherosclerosis, decreased elasticity of arteries, i.e.. increased "stiffness"

and progressive renal insufficiency, an argtunent can be made that bradycardia is another heretofore unrecognized important factor, one for which a new **treatment** option is available that will slow or prevent the progression of simple hypertension to the drug resistant variety and ultimately diastolic heart failure. Aging causes a drop in the heart rate. which is called sino atrial node dysfunction (SAND). SAND activates the sympathetic nervous system to increase peripheral vascular resistance according to the fluidic law below. The basic tenet of mammalian hemodynamics is that total blood flow is equal to driving pressure divided by resistance. This can be expressed in the same manner as Ohm's Law of electricity.

$$\Delta P = Q R$$

where R is resistance to flow, AP is the change in pressure across the circulation loop (systemic / pulmonary) from its beginning (immediately after exiting the left ventricle / right ventricle) to its end (entering the right atrium / left atrium), and Q is the flow through the vasculature (when discussing systemic vascular resistance (SVR) this is equal to cardiac output). This is the hydraulic version of Ohm's law, V=IR (which can be restated as R=V/I), in which the pressure differential is analogous to the electrical voltage drop, flow is analogous to electric current, and vascular resistance is analogous to electrical resistance. In some embodiments the algorithm processes SVR instead of SBP, DBP, or mean AP. The math is different, but the logic and functionality is essentially the same.

Consider first the nature of pathophysiology. If heart rate falls, mean arterial blood pressure can be maintained by increasing stroke volume (SV), which is a known compensatory mechanism of the healthy heart. However, when the left ventricle is impaired for a variety of reasons, such as prior heart attacks, hypertensive enlargement, primary muscle disease, or in the presence of significant valvar heart disease, and for other reasons, and HR also falls, R must increase to maintain blood pressure.

It follows, therefore, that the combination of both bradycardia (HR too low) and impaired SV would lead to increased R which would then lead to clinical hypertension and ultimately hypertensive heart failure when cardiac output is inadequate to overcome excess peripheral resistance.

It also follows that, in a patient with elevated R (significant hypertension) due to both reduced HR and SV, increasing HR, such as through the action of a pacemaker, would reduce R and thereby reduce the need for medication. Lowering R by this means should also result in a lower incidence of hypertension-induced heart failure.

If the heart fails, blood flow to the body's tissues must be maintained by a combination of increased left ventricular stroke volume (LVSV) and pulse rate (PR). A combination of aging, left ventricular hypertrophy due to hypertension, and other factors such as atherosclerosis progressively impair left ventricular performance which further demands an increase in PR. This negative feedback loop is worsened by increasing LV hypertrophy stimulated by increased LV work against high PR. Diastolic heart failure occurs when the higher filling pressure required by the stiff and hypertrophied left ventricular causes back up of blood into the lungs. This results in the classic clinical presentation of DCHF, which includes shortness of breath. fatigue, and peripheral edema. As the right and left atria stretch due to the effect of higher ventricular filling pressures, a compensatory mechanism is triggered within the atrial tissues to reduce PR and intravascular volume. Although it is not currently clearly understood, we believe that this compensatory mechanism includes the release of atrial naturetic peptides.

Turn now to clinical data supporting relevant to the illustrated embodiments of the invention. We studied retrospectively thirty patients satisfying the standard criteria for permanent pacemaker implant based upon bradycardia (sinus node dysfunction) who also had drug resistant hypertension. We hypothesized that if the above argument is true, the patient's hypertension post pacemaker implant should be lessened as manifested by lower blood pressure readings compared to pre-implant levels, and the need for hypertension medication lessened.

Thirty elderly patients (n = 30) with impaired SV bradycardia (sinoatrial node dysfunction,) who also had drug resistant hypertension (HTN) defined as the chronic use of at least four HTN agents from different drug classes not including a beta adrenergic blocking agent and who satisfied Class 1A criteria for permanent pacemaker implantation were studied before and after pacemaker implantation. A significant change in HTN management was defined as a drop of 15 mmHg systolic, or 10 mmHg diastolic on multiple readings over at least three months after pacemaker implant. and/or the deletion at least one anti-HTN agent without concomitant increase in the dosage of any other agent during the same period of time.

Six months after pacemaker implant 23 of 30 patients ( 77%, p<, 0.05 ) showed a significant improvement in HTN. 18 patients had a significant decrease in systolic pressure. 9 patients a significant decrease in both in both systolic and diastolic blood pressure, and 18 patients a change in drug usage: 19 patients dropped at least one antihypertensive drug, 12 patients dropped two drugs, and 2 patients dropped three drugs. The average onset of this effect was observed 1 +/- 0.6 months after pacemaker implant.

The data proves that, in elderly patients with the combination of impaired stroke, sinus node dysfunction satisfying AHA criteria for pacemaker implant, and drug resistant HTN, **pacemaker** implant may significantly improve HTN, and hypertension management. The impaired stroke volume of these patients was presumed volume based upon left ventricular hypertrophy and diastolic dysfunction which is impaired LV relaxation. Pacemaker implant should also improve the long-term outcomes of patients with significant drug resistant HTN and concomitant sinus node dysfunction by reducing not only the need for complex drug regimens, but also the development of the complications of drug resistant hypertension. including diastolic heart failure, kidney disease, and stroke.

The disclosed software should be added to AICD's which are not primary pacing devices and lack an RA lead when the patient has heart failure but not enough bradycardia to qualify for a pacemaker. This would result in single chamber ICDs being dropped in favor of dual chamber ICDs exclusively in patients with DRH and DRH with DCHF. While this is an attractive hypothesis, we currently have no clinical data on this group of patients (with systolic heart failure) and permanent pacing. The primary goal of pharmacological therapy in heart failure is to reduce R. Because such patients have a high incidence of the later development of sinus node dysfunction (Bigger JT Jr, Reiffel JA. Sick sinus syndrome. Annu Rev Med. 1979;30:91-118.) and are often administered drugs that suppress heart rate as a side effect of therapy, such as beta adrenergic blocking drugs, an argument can be made to implant a device that combines both an AICD and dual chamber function (with a third lead or CRT-D as appropriate) as the first device.

For the purpose of this application. bradycardia is defined as a mean heart rate sustained less than 60 beats per minute; chronotropic incompetence is defined as when HR fails to reach an arbitrary percentage (either 85%, 80%, or less commonly, 70%) of the age predicted maximal HR (usually based on the "220-age" equation) obtained during an incremental dynamic exercise test. We focused on patients with DRH and DRH with DCHF and found an improvement in DRH in terms of the number of drugs needed, actual lowering of SBP in both studies and DBP too in the other, and a reduction in hospitalizations for heart failure.

It can now be appreciated that the embodiments of the invention include various kinds of blood pressure sensing devices, such as non-invasive devices like cuffless wrist-type (non-pneumatic wave form analysis), cuff type arm or leg devices, cuffless waveform devices for BP analysis on any region of the body where arterial pulse can be sensed, e.g. using optical, plethysmographic, thermographic, electrical impedance, or electromagnetic means. Also included are invasive devices implanted in blood vessel outside the heart or implanted inside the heart.

The controlling software may be located in the blood pressure sensing device which then sends signal to pacemaker. in a peripheral device, but not the blood pressure sensor or the pacemaker, namely in a smart phone or computer, a conventional medical office e-programmer, an iPad (near the patient or a remote site), or in the pacemaker. The software is based on either manual input or is automatic. Control signals are generated based on measured systolic or diastolic BP, or mean blood pressure. The control signals are used to adjust right atrial pacing. up or down although the scope of the invention also extends to RV and LV pacing.

The pacemakers which are employed to implement the pacing control include atrial pacemakers (atrioventricular conduction intact, lead in RA), dual chamber pacemakers (atrioventricular conduction not intact, leads in RA and RV), bi-ventricular pacemakers (used in systolic heart failure where intraventricular conduction is prolonged), also known as a CRT, dual chamber AICD, (essentially a dual chamber pacemaker with a shocking lead in RV), and CRT-D, (a bi-ventricular pacemaker with a shocking lead in RV).

Consider three embodiments. The simplest or most primitive example is an open loop, manually controlled system. It is employed as a medical office procedure, uses a standard pneumatic blood pressure cuff, a doctor's office pacemaker programmer near the patient, and a conventional normally programmable dual chamber pacemaker. The patient sits near doctor. Blood pressure is taken with standard pneumatic cuff. The physician looks for systolic blood pressure on a printed table displaying the disclosed algorithm in a tabular format and determines an optimum RA pacing rate. The physician places a pacemaker programmer wand (RF source) over the patient's pacemaker and uses the programmer to reprogram the pacemaker to desired RA pacing rate according to the teachings of the disclosed embodiments.

The next preferred embodiment is configured as a closed loop, automatic system. A cuffless wrist-type blood pressure sensor with wireless connectivity is used and a smart phone app with disclosed algorithm receives blood pressure readings. The BP reading is encrypted and sent to the pacemaker wirelessly. The pacemaker receives the encrypted blood pressure reading, authenticates, and alters RA pacing rate.

The third embodiment is a home monitoring and remote processing system. All front-line pacemakers offer home monitoring. The patient is given a device that is commonly left at the bedside. When the patient moves near it, such as going to bed, the device wirelessly interrogates the pacemaker, monitoring such things as battery voltage and recent arrhythmia activity, and sends it via the phone lines in encrypted form to a remote central station operated by the pacemaker company. Inbound instructions are sent by the same system to alter the pacemaker's programming and/or alert the treating physician that adverse events have occurred, such as arrhythmias or device dysfunction.

In additional ones of the illustrated embodiments all different types of pacemakers, e.g. AP, DCP, D-C/AICD. CRT, or CRT-D, were operated or used for a wide permutation of cardiac symptoms or abnormalities, for example for the indicated combinations:

DRH + B +ISV-S

DRH + B + ISV-D

DRH + CI + ISV-S

DRH + CI + ISV-D

DRH + B

$$DRH + CI$$

$$DRH + CI$$

$$DRH + DHF + B \text{ (This adds the variable of DHF also being present)}$$

$$DRH + DHF + CI$$

$$DRH + SHF + B \text{ (This adds the variable of SHF also being present.)}$$

$$DRH + SHF + CI$$

Where: DRH = Drug Resistant Hypertension; B = Bradycardia defined as a persistent HR < 60 (higher than the Guidelines); CI = chronotropic incompetence as defined above; ISV-S = Impaired Stroke Volume due to failure of systolic function; ISV-D = Impaired Stroke Volume due to failure of diastolic function; DHF = diastolic heart failure or heart failure with a preserved LV function (LVEF > 50%). This is another form of ISV where the Left Ventricle contracts normally, but *relaxes* in an impaired manner: SHF = systolic heart failure or heart failure with reduced LV function (LVEF < 35%);
The illustrated embodiments of the invention were also directed to methods of operating or using a pacemaker t to treat heart failure (systolic and diastolic) by reducing peripheral resistance (R) with or without the presence of sinus node dysfunction. The current guidelines for the implantation of a permanent pacemaker in the presence of sinus node dysfunction are too strict in patients who also have heart failure. Many heart failure patients have lower than effective heart rates (relative bradycardia) and both with and without high resistance, but do not satisfy the very strict current AHA Guidelines for pacemaker implant. The presence of relative bradycardia sufficient to increase peripheral resistance (the primary therapeutic point of attack for non-surgical heart failure therapy) and heart failure should be sufficient to warrant pacemaker implant. This would include patients with relative bradycardia and lesser chronotropic incompetance not currently meeting the AHA Guidelines for pacemaker implant.

The magnitude of the diminished capacity to respond to Bradycardia is proportional to LV function. The lower the LV function, the more profound is the effect of bradycardia on peripheral resistance. which is manifest as elevated blood pressure, and peripheral resistance is the major determinant of morbidity and mortality in heart failure. Therefore, the criteria for pacemaker implant in patients who have DRH and DRH with DCHF, and all forms of CHF and **relative** bradycardin should not be limited by the current AHA Guidelines. This increases the cohort of patients who should have atrioventricular pacing to include all patients with heart failure and relative bradycardia. While relative bradycardia is defined the purposes of this embodiment as either the presence of chronotropic incompetence or a HR less than 60 and greater than 40, it is to be expressly understood that this definition can be modified as determined by later clinical trials of the pacing methodology without departing from the scope of the invention.

Thus, the illustrated embodiments of the invention are directed to a method of operating or using an implantable pacemaker (AP, DCP, CRT, CRT-D) to treat diastolic heart failure in patients with concomitant bradycardia relative or meeting the AHA guidelines, concomitant chronotropic incompetence and/or chronotropic incompetence with drug resistant hypertension to optimize peripheral resistance by the restoration of a normal heart rate. These methods of operation and use of implanted pacemakers are based upon utilizing a pacing therapy to reduce peripheral resistance as means of treatment of at least some types of heart failure. Reducing peripheral resistance is the primary goal of nonsurgical heart failure treatment, surgical treatment includes bypass surgery, heart transplantation, and implantation of heart assist devices.

Therefore, it can be appreciated that the operation and use of implanted pacemakers to treat heart failure is the primary end point of the pacing operation or use. The endpoints of treatment are both heart failure and concomitant drug resistant hypertension. In each permutation of systems what is indicated is the use and operation of all types of pacemakers, for example including in such diagnostic permutations as: DHF + CI; DHF + B; SHF + B; and SHF + CI as **well as in** the treatment of heart failure with concomitant drug resistant hypertension including in such diagnostic permutations as: SHF + **B** +DRH; SHF + CI + DRH; DHF + B + DRH; and DHF + CI + DRH.

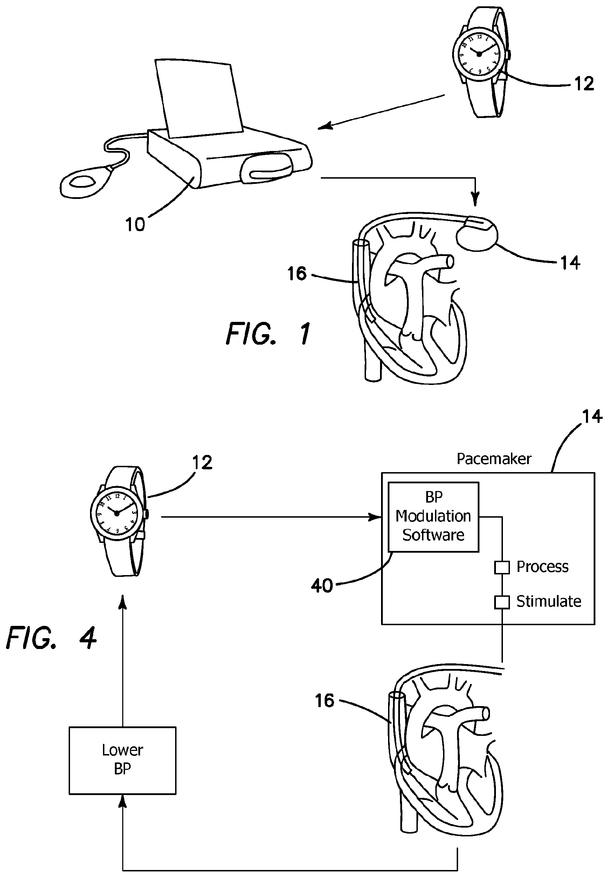

Consider an embodiment **of the** invention wherein **it is realized** in a scenario as shown in Fig. 1 with an external programming device 10 and a wristwatch type blood pressure monitor 12. The patient is fitted with a pacemaker 14 connected to the patient's heart 16 with the **blood** pressure modulation software resident in the pacemaker's processor. The patient is also fitted the **blood** pressure monitor 12 mounted in a wristwatch band with encrypted blue tooth connectivity linking it uniquely to the patient's pacemaker 14. The patient or clinician activates wristwatch blood pressure monitor 12 and selects the number of blood pressure readings to store, and how far apart in minutes the measurements are separated. This data set comprises the baseline blood pressure readings of the illustrated embodiment of the invention. The BP measurements are carried out according to the predetermined schedule and the data set, the baseline blood pressure readings, is created and stored in the pacemaker 14. Using the external programming device 10, the clinician pairs the patient's wristwatch blood pressure monitor 12 to the pacemaker 14 by entering the unique serial numbers of the pacemaker 14 and the blood pressure monitor 12 allowing blue tooth encrypted interconnectivity of both devices. If the blood pressure monitor 12 is inactivated for any reason, the blood pressure algorithm in the pacemaker 14 becomes dormant and the pacemaker 14 returns to regular function unmodulated by the external blood pressure readings or the internal blood pressure modulation algorithm.

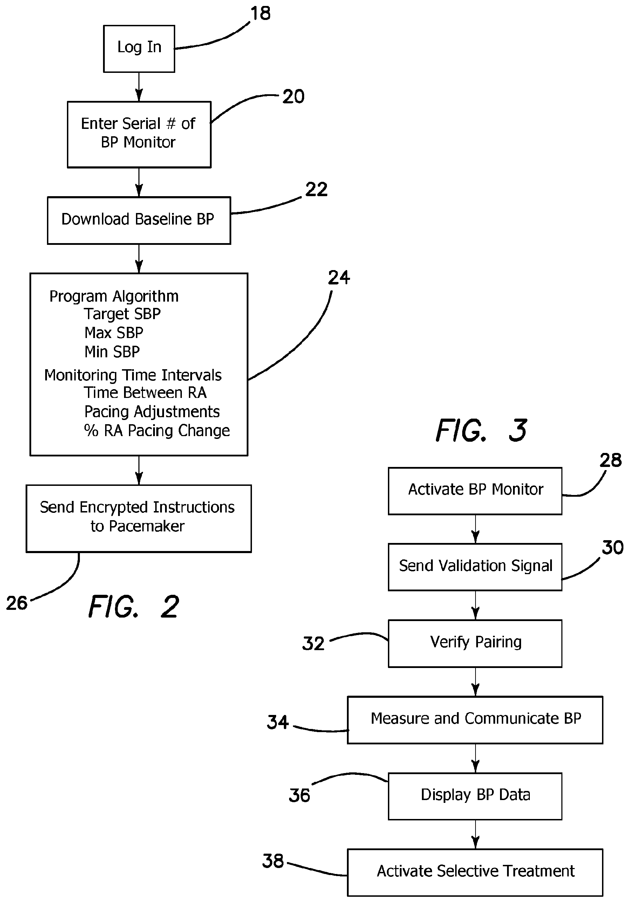

The clinician inputs the following parameters through the external programming device 10, which parameters are transmitted to the pacemaker 14 and integrated into the blood pressure regulating algorithm. The clinician inputs a desired SBP (systolic blood pressure) and inputs a lower limit of acceptable SBP. For example, the desired treatment interval in minutes and a desired RA pacing change per treatment interval from 0 - 40% is input. After the clinician directed software functions are programmed, the external programming device 10 is inactivated and the patient's pacemaker 14 paired with the wearable blood pressure monitor 12 begins automatic functioning. The flow diagram of Fig.2 summarizes this initial programming session. At step 18 the clinician logs onto the external programming device 10 to gain access to the pacemaker 14 and BP monitor 12. He or she enters the serial number of patient's wearable blood pressure monitor 12 at step 20. The pacemaker 14 will now only accept data from the designated BP monitor 12. The clinician downloads into the external programming device 10 the baseline BP data set from the patient's BP monitor 12 at step 22. The clinician uses that data set to program the treatment algorithm by setting at step 24: the target SBP; maximal SBP; minimum SBP; selected time between monitor intervals, which may be preset at ten minutes; selected time between RA pacing adjustments, which may be preset at ten minutes; and percentage RA pacing change per treatment interval, which may be preset at 5%. The instructions are encrypted and sent to the pacemaker 14 at step 26.

As shown in the flow diagram of Fig. 3 the blood pressure monitor 12 and the patient's pacemaker 14 are paired using encrypted blue tooth technology. The patient activates the wearable BP monitor's 12 encrypted blue tooth link to the pacemaker 14 at step 28. BP monitor 12 sends a test signal to pacemaker 14 as step 30 to validate pairing and integrity of the signal. The monitor 12 notifies the patient that encrypted pairing is complete at step 32. BP monitor 12 measures the BP. The BP data is encrypted and communicated to the external programming device 10 and to the pacemaker 14 at step 34. The external programming device 10 decrypts the data and displays it at step 36. Selective treatment by pacemaker 14 is then activated through the external programming device 10 at step 38.

While the patient carries on ordinary activities, the software in the pacemaker 14 processes the blood pressure data transmitted by the blood pressure monitor 12 and establishes: 1) the steady state BP, namely the average blood pressure based upon recent blood pressure readings; and 2) the steady state RA pacing, namely the average right atrial pacing rate during the same time intervals.

If the average systolic blood pressure, namely most recent steady-state readings, exceeds the preprogrammed limits set by the clinician, treatment is automatically initiated. If the most recent steady state readings are below the treatment plateau programmed by the clinician, the software does not alter the right atrial pacing rate and no blood pressure treatment is delivered. If a decision to treat has been made by the software, the RA pacing rate is increased by either the default level of 5% per treatment, or by a different value pre-programmed by the clinician using the external programming device 10. For example one possible treatment option would be to increase RA pacing 7%

The SBP is monitored for twenty minutes, or for a different time interval preprogrammed by the clinician, to establish the new blood pressure baseline. If the SBP is still above the clinician's pre-selected optimal SBP, the treatment is repeated by increasing the RA pacing rate another 5% or by a different amount as pre-programmed by the clinician. This cycle of monitoring and selective treatment is repeated until either the SBP falls to the pre-programmed optimal level, or the RA pacing rate exceeds 40% or a different maximal value pre-programmed by the clinician.

Fig. 4 illustrates the selective treatment in a rate modulation mode. The blood pressure monitor 12, the blood pressure modulating software 40, and the pacemaker 14 form an automatic feedback loop to regulate pacemaker function, which in this embodiment is RA pacing but need not be so limited, to lower blood pressure. The algorithm utilizes the following parameters to determine or modulate the optimal RA pacing percentage to optimize BP:

1) Blood pressure input, both systolic and diastolic, from an external blood pressure monitor 12.

2) RA pacing percentage

3) Instantaneous heart rate

4) Maximal preset RA pacing percentage, which is set at 40% unless overridden by further data collection or the clinician.

5) Desired maximum blood pressure, most likely systolic. Insufficient data exists at this time to determine the relationship between diastolic BP and RA pacing.

6) Desired minimum blood pressure.

7) Sampling time between blood pressure measurements, namely the interval increase or decrease in RA pacing during each treatment Interval.

**8)** The number of BP measurements necessary to calculate steady state BP

**9)** The time between BP measurements to calculate steady state BP

**10)** The time in minutes between treatment Intervals.

The treatment algorithm in the rate modulation mode relates the instantaneous change in SBP with the change in RA pacing percentage, such that the drop in SBP is mapped to an increase in RA pacing times a constant A + constant B.

$$\Delta SBP = (\Delta RA) A + B$$

In the present embodiment based upon currently available clinical data, which may be later refined by subsequent data gathered, A = -0.31, and B = 16. In the currently preferred embodiment of the treatment algorithm the change in SBP and RA pacing are expressed as a percentage.

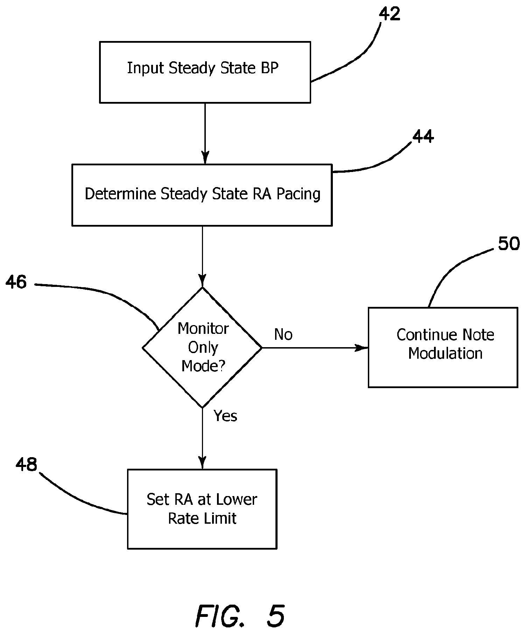

Fig. 5 is a flow diagram which illustrates further processing undertaken in pacemaker 14. At step 42 steady state BP is input, either baseline BP if it is the first use, the BP during the last ten minutes, or such time as otherwise programmed by clinician. At step 44 access steady state RA pacing, either baseline if it is a first use, the rate during the last ten minutes, or at such time otherwise as programmed by clinician. If the software is in monitor-only mode as determined at step 46, either because it has been disabled or the desired blood pressure has been detected in steady state, the RA pacing rate is set at step 48 by the clinician at a selected lower rate limit. Otherwise pacemaker 14 is or will continue to operate in the rate modulation mode.

When the patient exercises, or the pacemaker's rate modulation software is activated for any reason, BP will be affected and RA pacing will rise. The algorithm will default to monitor-only mode so long as the increase in RA pacing does not drop the blood pressure below the preset minimum SBP. If the SBP drops below the preset minimum, the rate modulation mode will be inhibited. This is a new pacemaker safeguard for all devices across all brands that offer rate modulation software as feature of their pacemakers. It will protect the patient from an excessive drop in SBP caused by excessive RA pacing, or dual chamber pacing such as RAIRV or RAIL V.

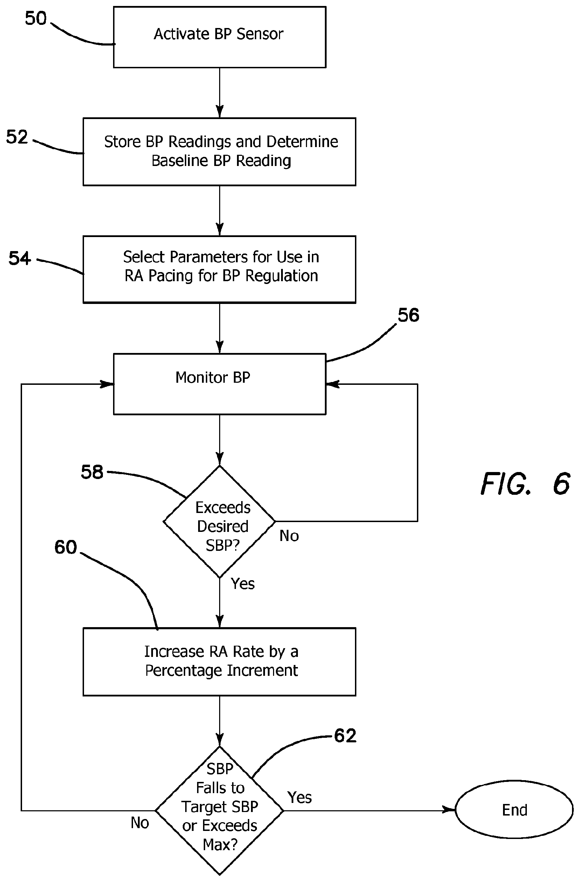

The apparatus and a method operate a pacing device 14 as depicted diagrammatically in the flow diagram of Fig. 6 by including the steps of:

**1)** activating a wristwatch blood pressure sensor 12 at step 50;

**2)** storing a number of blood pressure readings, temporarily separated in a predetermined pattern, to establish a baseline blood pressure reading at step 52;

**3)** selecting at step 54 the following parameters for use in the right atrial pacemaker for blood pressure regulation;

**i.** desired SBP (systolic blood pressure);

**ii.** lower limit of acceptable SBP;

**iii.** desired treatment interval in minutes; and/or

**iv.** desired RA pacing change per treatment interval where for example the change ranges from 0 - 40% or such other predetermined percentage range consistent with the teachings of this invention;

**4)** monitoring blood pressure at step 56 and if blood exceeds the desired SBP as determined at step 58, using the pacemaker 14 to treat the patient at step 60 by:

**i.** increasing the RA pacing rate by either a default level of 5% per treatment, or by a different predetermined value;

**ii.** monitoring the SBP for a predetermined time period to establish the new blood pressure baseline;

**iii.** comparing SBP to a pre-selected optimal SBP:

**iv.** increasing the RA pacing rate by a predetermined incremental amount; and

**v.** repeating the steps of comparing SBP and increasing RA pacing rate until either the SBP falls to the target or pre-programmed optimal level, or the RA pacing rate exceeds a predetermined maximal value as depicted at step 62.

The algorithm and non-claimed methods of utilization herein described also significantly improve existing pacemaker heart rate modulating programs by optimizing RA pacing in response to blood pressure as well as exercise parameters. Exercise-induced syncope (fainting) or dizziness is a well-recognized clinical phenomenon. When a patient with a pacemaker exercises, his/her heart rate will be regulated by the pacemaker's programmed rate modulation software if activated. According to the data herein presented, elevation of the patient's heart rate could also result in a lowering of blood pressure such that the patient might experience dizziness or syncope. By use of this methodology in the patient's pacemaker an excessive drop in blood pressure is prevented by inhibition of the patient's pacemaker's rate modulation function.

**Claims**

1. A system suitable for operating a pacemaker (14) comprising:

   a blood pressure monitor (12) couplable to a patient;
   a memory communicated to the monitor for storing a number of blood pressure readings;
   a processor communicated to the memory for determining a baseline blood pressure reading;

   the system being **characterized in that** the processor determines the following parameters to use to control the pacemaker (14) for blood pressure regulation: a target BP, blood pressure, a lower limit of acceptable BP; a target treatment interval in minutes and/or target pacing rate change per treatment interval at a predetermined pacing rate change percentage;
   wherein the processor determines, while monitoring blood pressure, if blood pressure exceeds the target BP, wherein the pacemaker (14) has a controllable pacing rate to treat the patient by: increasing the pacing rate of the pacemaker by either a default level of 5% per treatment, or by a different predetermined value;
   wherein the processor monitors the BP for a predetermined time period to establish the new blood pressure baseline; compares BP to a preselected optimal BP; increasing the pacing rate of the pacemaker (14) by a predetermined incremental amount; and repeats the steps of comparing BP and increasing the pacing rate of the pacemaker (14) until either the BP falls to the target BP, or the pacing rate of the pacemaker (14) exceeds a predetermined maximal value.

2. The system of claim 1, where the pacing rate of the pacemaker (14) is a RA pacing rate.

3. The system of claim 1 or 2, where the processor monitors peripheral blood pressure, intravascular blood pressure or intracardiac blood pressure.

4. The system of claim 3, where the processor monitors peripheral blood pressure with a pneumatic device, a non-pneumatic device or an implantable device implanted within a blood vessel or a heart chamber.

5. The system of any of the preceding claims, where the processor also monitors blood oxygen levels, glucose levels, blood electrolytes levels or other blood parameters and controlling the pacing rate in response to the monitored blood oxygen levels, glucose levels, blood electrolytes levels or other blood parameters.

6. The system of any of the preceding claims,

   where the pacing device is a RA pacemaker (14),
   where the processor determines a percentage of time paced in the RA to auto-adjust pacing rate changes at determined minimal and maximal increments, and a maximal paced HR limit established for each patient, not to be exceeded by the pacing device irrespective of BP changes,
   where if blood pressure exceeds the target BP, the pacemaker (14) is selected to having a RA pacing rate to treat the patient,
   where the processor treats the patient by increasing the RA pacing rate of the pacemaker by either a default level of 5% per treatment, or by a different predetermined value,
   where the processor increases the pacing rate of the pacemaker (14) by a predetermined incremental amount increases the RA pacing rate and
   where the processor repeats the steps and compares BP and increases the RA pacing rate of the pacemaker (14) until either the BP falls to the target BP, or the RA pacing rate of the pacemaker (14) exceeds a predetermined maximal value.

7. The apparatus of any of the preceding claims , where the blood pressure monitoring device (12) is wrist mounted, an arm cuff or an implantable sensor.

8. The apparatus of any of the preceding claims, where the blood pressure monitoring device (12) communicates wirelessly with the pacemaker.

9. The apparatus of any of the preceding claims, where the blood pressure monitoring device (12) further comprises a pulse oximeter, and/or a chemical sensor.

10. The apparatus of any of the preceding claims, where the blood pressure monitoring device (12) monitors systolic blood pressure.

11. The apparatus of any of the preceding claims, where the blood pressure monitoring device (12) monitors diastolic blood pressure.

12. The apparatus of any of the preceding claims, where the implantable pacemaker (14) is implantable at a vascular site or a cardiac site.

**Patentansprüche**

1. System zum Betreiben eines Schrittmachers (14), Folgendes umfassend:

ein Blutdruckmessgerät (12) zum Anschließen an einen Patienten;
einen mit dem Messgerät verbundenen Speicher zum Speichern einer Anzahl von Blutdruckmesswerten;
einen mit dem Speicher verbundenen Prozessor zum Bestimmen eines Basis-Blutdruckmesswerts; wobei das System **dadurch gekennzeichnet ist, dass** der Prozessor die folgenden Parameter bestimmt, die zum Steuern des Schrittmachers (14) für eine Blutdruckregulierung zu verwenden sind: einen Ziel-Blutdruck, eine Untergrenze eines akzeptablen Blutdrucks; ein Ziel-Behandlungsintervall in Minuten und/oder eine Ziel-Schrittmacherfrequenzänderung pro Behandlungsintervall mit einem vorbestimmten Prozentsatz der Schrittmacherfrequenzänderung;
wobei der Prozessor während des Überwachens des Blutdrucks feststellt, ob der Blutdruck den Ziel-Blutdruck überschreitet,
wobei der Schrittmacher (14) eine steuerbare Schrittmacherfrequenz aufweist, um den Patienten durch Folgendes zu behandeln: Erhöhen der Schrittmacherfrequenz des Schrittmachers entweder um einen Standardwert von 5 % pro Behandlung oder um einen anderen vorgegebenen Wert;
wobei der Prozessor den Blutdruck über einen vorbestimmten Zeitraum überwacht, um den neuen Basis-Blutdruckwert festzulegen; den Blutdruck mit einem vorgewählten optimalen Blutdruck vergleicht; die Schrittmacherfrequenz des Schrittmachers (14) um einen vorbestimmten inkrementellen Betrag erhöht; und die Schritte des Vergleichens des Blutdrucks und des Erhöhens der Schrittmacherfrequenz des Schrittmachers (14) wiederholt, bis entweder der Blutdruck auf den Ziel-Blutdruck fällt oder die Schrittmacherfrequenz des Schrittmachers (14) einen vorbestimmten Maximalwert überschreitet.

2. System nach Anspruch 1, wobei die Schrittmacherfrequenz des Schrittmachers (14) eine RA-Schrittmacherfrequenz ist.

3. System nach Anspruch 1 oder 2, wobei der Prozessor den peripheren Blutdruck, den intravaskulären Blutdruck oder den intrakardialen Blutdruck überwacht.

4. System nach Anspruch 3, wobei der Prozessor den peripheren Blutdruck mit einer pneumatischen Vorrichtung, einer nicht-pneumatischen Vorrichtung oder einer implantierbaren Vorrichtung, die in ein Blutgefäß oder eine Herzkammer implantiert ist, überwacht.

5. System nach einem der vorhergehenden Ansprüche, wobei der Prozessor auch Blutsauerstoffgehalt, Glukosespiegel, Blutelektrolytspiegel oder andere Blutparameter überwacht und die Schrittmacherfrequenz in Abhängigkeit von dem überwachten Blutsauerstoffgehalt, Glukosespiegel, Blutelektrolytspiegel oder anderen Blutparametern steuert.

6. System nach einem der vorhergehenden Ansprüche,

wobei die Schrittmachervorrichtung ein RA-Schrittmacher (14) ist,
wobei der Prozessor einen Prozentsatz der Zeit bestimmt, die in der RA zum automatischen Anpassen der Schrittmacherfrequenz-Änderungen bei bestimmten minimalen und maximalen Inkrementen verbracht wird, sowie einen für jeden Patienten festgelegten maximalen Herzfrequenz-Grenzwert, der von der Schrittmachervorrichtung unabhängig von Blutdruckänderungen nicht überschritten werden darf,

wobei, wenn der Blutdruck den Ziel-Blutdruck überschreitet, der Schrittmacher (14) so eingestellt wird, dass er eine RA-Schrittmacherfrequenz aufweist, um den Patienten zu behandeln,

wobei der Prozessor den Patienten behandelt, indem er die RA- Schrittmacherfrequenz des Schrittmachers entweder um einen Standardwert von 5 % pro Behandlung oder um einen anderen vorgegebenen Wert erhöht,

wobei der Prozessor die Schrittmacherfrequenz des Schrittmachers (14) um einen vorbestimmten inkrementellen Betrag erhöht, der die RA-Schrittmacherfrequenz erhöht, und

wobei der Prozessor die Schritte wiederholt und den Blutdruck vergleicht und die RA-Schrittmacherfrequenz des Schrittmachers (14) erhöht, bis entweder der Blutdruck auf den Ziel-Blutdruck fällt oder die RA-Schrittmacherfrequenz des Schrittmachers (14) einen vorgegebenen Maximalwert überschreitet.

7. Vorrichtung nach einem der vorhergehenden Ansprüche, wobei die Blutdrucküberwachungsvorrichtung (12) am Handgelenk befestigt ist, eine Armmanschette oder ein implantierbarer Sensor ist.

8. Vorrichtung nach einem der vorhergehenden Ansprüche, wobei die Blutdrucküberwachungsvorrichtung (12) drahtlos mit dem Schrittmacher kommuniziert.

9. Vorrichtung nach einem der vorhergehenden Ansprüche, wobei die Blutdrucküberwachungsvorrichtung (12) ferner ein Pulsoximeter und/oder einen chemischen Sensor umfasst.

10. Vorrichtung nach einem der vorhergehenden Ansprüche, wobei die Blutdrucküberwachungsvorrichtung (12) den systolischen Blutdruck überwacht.

11. Vorrichtung nach einem der vorhergehenden Ansprüche, wobei die Blutdrucküberwachungsvorrichtung (12) den diastolischen Blutdruck überwacht.

12. Vorrichtung nach einem der vorhergehenden Ansprüche, wobei der implantierbare Schrittmacher (14) an einer vaskulären Stelle oder an einer kardialen Stelle implantierbar ist.

**Revendications**

1. Système adapté pour faire fonctionner un stimulateur cardiaque (14) comprenant :

un moniteur de pression artérielle (12) pouvant être couplé à un patient ;
une mémoire en communication avec le moniteur pour le stockage d'un certain nombre de lectures de pression artérielle ;
un processeur en communication avec la mémoire pour la détermination d'une lecture de pression artérielle de référence ;
le système étant **caractérisé en ce que** le processeur détermine les paramètres suivants à utiliser pour commander le stimulateur cardiaque (14) pour la régulation de la pression artérielle : une BP, pression artérielle, cible, une limite inférieure de BP acceptable ; un intervalle de traitement cible en minutes et/ou un changement de fréquence de stimulation cible par intervalle de traitement à un pourcentage de changement de fréquence de stimulation prédéterminé ;
dans lequel le processeur détermine, tout en surveillant la pression artérielle, si la pression artérielle dépasse la BP cible,
dans lequel le stimulateur cardiaque (14) a une fréquence de stimulation pouvant être commandée pour traiter le patient par : augmentation de la fréquence de stimulation du stimulateur cardiaque soit par un niveau par défaut de 5 % par traitement, soit par une valeur prédéterminée différente ;
dans lequel le processeur surveille la BP pendant une période de temps prédéterminée pour établir la nouvelle référence de pression artérielle ; compare la BP à une BP optimale présélectionnée ; augmente la fréquence de stimulation du stimulateur cardiaque (14) d'une quantité incrémentielle prédéterminée ; et répète les étapes de comparaison de la BP et d'augmentation de la fréquence de stimulation du stimulateur cardiaque (14) jusqu'à ce que soit la BP tombe à la BP cible, soit la fréquence de stimulation du stimulateur cardiaque (14) dépasse une valeur maximum prédéterminée.

2. Système selon la revendication 1, dans lequel la fréquence de stimulation du stimulateur cardiaque (14) est une fréquence de stimulation RA.

3. Système selon la revendication 1 ou 2, dans lequel le processeur surveille la pression artérielle périphérique, la pression artérielle intravasculaire ou la pression artérielle intracardiaque.

4. Système selon la revendication 3, dans lequel le processeur surveille la pression artérielle périphérique avec un dispositif pneumatique, un dispositif non pneumatique ou un dispositif implantable implanté à l'intérieur d'un vaisseau sanguin ou d'une chambre cardiaque.

5. Système selon l'une quelconque des revendications précédentes, dans lequel le processeur surveille également les taux d'oxygène dans le sang, les taux de glucose, les taux d'électrolytes dans le sang ou d'autres paramètres sanguins et commande la fréquence de stimulation en réponse aux taux d'oxygène dans le sang, taux de glucose, taux d'électrolytes dans le sang ou autres paramètres sanguins surveillés.

6. Système selon l'une quelconque des revendications précédentes,

dans lequel le dispositif de stimulation est un stimulateur cardiaque RA (14),
dans lequel le processeur détermine un pourcentage de temps stimulé dans la RA pour régler automatiquement des changements de fréquence de stimulation à des incréments minimums et maximums déterminés, et une limite HR stimulée maximum établie pour chaque patient, qui ne doit pas être dépassée par le dispositif de stimulation indépendamment des changements de BP,
dans lequel si la pression artérielle dépasse la BP cible, le stimulateur cardiaque (14) est sélectionné pour avoir une fréquence de stimulation RA pour traiter le patient,
dans lequel le processeur traite le patient par augmentation de la fréquence de stimulation RA du stimulateur cardiaque soit par un niveau par défaut de 5 % par traitement, soit par une valeur prédéterminée différente,
dans lequel le processeur augmente la fréquence de stimulation du stimulateur cardiaque (14) d'une quantité incrémentielle prédéterminée augmente la fréquence de stimulation RA et
dans lequel le processeur répète les étapes et compare la BP et augmente la fréquence de stimulation RA du stimulateur cardiaque (14) jusqu'à ce que soit la BP tombe à la BP cible, soit la fréquence de stimulation RA du stimulateur cardiaque (14) dépasse une valeur maximum prédéterminée.

7. Appareil selon l'une quelconque des revendications précédentes, dans lequel le dispositif de surveillance de la pression artérielle (12) est monté au poignet, un brassard ou un capteur implantable.

8. Appareil selon l'une quelconque des revendications précédentes, dans lequel le dispositif de surveillance de la pression artérielle (12) communique sans fil avec le stimulateur cardiaque.

9. Appareil selon l'une quelconque des revendications précédentes, dans lequel le dispositif de surveillance de la pression artérielle (12) comprend en outre un oxymètre de pouls et/ou un capteur chimique.

10. Appareil selon l'une quelconque des revendications précédentes, dans lequel le dispositif de surveillance de la pression artérielle (12) surveille la pression artérielle systolique.

11. Appareil selon l'une quelconque des revendications précédentes, dans lequel le dispositif de surveillance de la pression artérielle (12) surveille la pression artérielle diastolique.

12. Appareil selon l'une quelconque des revendications précédentes, dans lequel le stimulateur cardiaque implantable (14) est implantable au niveau d'un site vasculaire ou d'un site cardiaque.

FIG. 1

FIG. 4

FIG. 2

Log In — 18

Enter Serial # of BP Monitor — 20

Download Baseline BP — 22

Program Algorithm
Target SBP
Max SBP
Min SBP

Monitoring Time Intervals
Time Between RA
Pacing Adjustments
% RA Pacing Change

— 24

Send Encrypted Instructions to Pacemaker

26

FIG. 3

Activate BP Monitor — 28

Send Validation Signal — 30

32 — Verify Pairing

34 — Measure and Communicate BP

36 — Display BP Data

38 — Activate Selective Treatment

FIG. 5

50 — Activate BP Sensor

52 — Store BP Readings and Determine Baseline BP Reading

54 — Select Parameters for Use in RA Pacing for BP Regulation

56 — Monitor BP

58 — Exceeds Desired SBP?

No

Yes

60 — Increase RA Rate by a Percentage Increment

62 — SBP Falls to Target SBP or Exceeds Max?

No

Yes

End

*FIG. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160220824 A1

- US 2009036940 A1

**Non-patent literature cited in the description**

- **BIGGER JT JR** ; **REIFFEL JA**. Sick sinus syndrome. *Annu Rev Med.*, 1979, vol. 30, 91-118